# FASCICULE DE BREVET EUROPEEN

(11) **EP 3 406 289 B1**
(45) Date de publication et mention de la délivrance du brevet: **11.03.2020**
(21) Numéro de dépôt: 18169452.2
(22) Date de dépôt: 26.04.2018
(51) Int. Cl.: A61M 16/16, A61M 16/10

(54) **RÉSERVOIR D'EAU AMÉLIORÉ POUR UN HUMIDIFICATEUR DE GAZ CHAUFFANT ALIMENTÉ PAR UN VENTILATEUR MÉDICAL**
VERBESSERTER WASSERBEHÄLTER FÜR EINEN BEHEIZTEN GASBEFEUCHTER, DER DURCH EIN MEDIZINISCHES BEATMUNGSGERÄT GESPEIST WIRD
IMPROVED WATER TANK FOR A HEATING GAS HUMIDIFIER SUPPLIED BY A MEDICAL VENTILATOR

(30) Priorité: 24.05.2017 FR 1754590
(43) Date de publication de la demande: 28.11.2018
(73) Titulaire: Air Liquide Medical Systems, 92160 Antony (FR)
(72) Inventeur: LEBATTEUR, Nicolas, 91220 BRETIGNY SUR ORGE (FR); MOVSCHIN, Antoine, 92160 Antony (FR); GUIDUCCI, Hadrien, 75020 PARIS (FR)
(74) Mandataire: Pittis, Olivier

(56) Documents cités:
- EP-A1- 2 540 335
- EP-A1- 2 848 277
- EP-A2- 1 055 431
- WO-A1-2014/038968
- WO-A1-2014/138804
- DE-A1- 10 226 160
- US-A1- 2013 174 843

## Description

L'invention concerne un réservoir à eau amélioré pour un dispositif humidificateur chauffant servant à l'humidification des gaz issus d'un appareil d'assistance respiratoire médical, ledit réservoir à eau comprenant un couvercle remplissant une fonction d'anti-éjection d'eau, en particulier lorsque le réservoir est en position inclinée, ainsi qu'un humidificateur de gaz intégrant un tel réservoir et un ensemble humidificateur / ventilateur médical.

Habituellement, un dispositif d'humidification de gaz chauffant, généralement appelé humidificateur, comprend un réservoir amovible destiné à contenir un volume d'eau. Ce réservoir est configuré pour permettre le passage d'un débit d'air entre un orifice d'entrée et un orifice de sortie. Son fond comporte une plaque métallique destiné à transmettre à l'eau, de la chaleur provenant d'un boitier chauffant, dans lequel vient se loger le réservoir. Le fond du boitier comporte un élément chauffant relié à ou en contact avec une autre plaque métallique dont le rôle est de transmettre la chaleur produite par l'élément chauffant du boitier à la plaque métallique du réservoir, par l'intermédiaire de la seconde plaque métallique.

Pendant le fonctionnement de l'humidificateur, l'air provenant d'un appareil médical d'assistance ventilatoire, couramment appelé «ventilateur», entre dans le réservoir, s'y réchauffe et s'y charge en humidité grâce à l'eau qui est chauffée dans le réservoir. L'air chaud humidifié est ensuite acheminé jusqu'aux voies respiratoires d'un patient, via notamment un conduit de gaz flexible alimentant une interface respiratoire, tel un masque respiratoire ou analogue.

Les documents EP-A-2540335, WO-A-2014/138804 et EP-A-2848277 enseignent des cuves à eau pour humidificateur de gaz, à couvercle avec orifice d'entrée de gaz en communication fluidique avec un élément de conduit se projetant dans le volume interne de la cuve et muni d'une extrémité libre munie d'une bouche de sortie de gaz.

Toutefois, l'utilisation, par un patient à domicile, d'un humidificateur chauffant, contenant un réservoir rempli d'eau, en complément d'un ventilateur pulmonaire, provoque souvent des cas de défaut liés à une éjection d'eau en dehors de l'humidificateur, c'est-à-dire une sortie non voulue d'eau du réservoir. En effet, sous l'effet des débits d'air induits par le ventilateur ou par le patient, il peut arriver que de l'eau reflue vers l'entrée ou vers la sortie du réservoir de l'humidificateur.

Le cas où de l'eau est éjectée vers l'entrée du réservoir de l'humidificateur, c'est-à-dire vers le ventilateur, est particulièrement problématique car l'eau éjectée peut endommager la micro-soufflante électrique, encore appelée turbine ou compresseur, délivrant le flux d'air et donc détériorer le ventilateur médical.

Le cas où l'eau est éjectée vers la sortie, c'est-à-dire vers le circuit relié au patient, est moins critique, bien qu'il puisse présenter une gêne importante pour le patient et nuire au succès de son traitement par inhalation de gaz.

Le problème qui se pose est dès lors d'éviter ou de limiter l'éjection d'eau en dehors du réservoir de l'humidificateur de manière à réduire le risque de détérioration de la micro-soufflante électrique du ventilateur médical alimentant l'humidificateur en gaz respiratoire à humidifier.

La solution de l'invention concerne alors un réservoir pour humidificateur de gaz chauffant servant à l'humidification de gaz issu d'un appareil d'assistance respiratoire médical, typiquement un ventilateur médical, comprenant une cuve à eau comprenant une paroi périphérique et un fond muni d'une plaque métallique, et un couvercle agencé sur la cuve et solidaire de la paroi périphérique de ladite cuve, le couvercle comprenant en outre un orifice d'entrée de gaz en communication fluidique avec un élément de conduit, l'élément de conduit se projetant dans le volume interne de la cuve en direction de la paroi périphérique de ladite cuve, et comprenant une extrémité libre munie d'une bouche de sortie, caractérisé en ce que :
- le couvercle comprend en outre une chambre de sortie de gaz délimitée par la paroi externe du couvercle et une paroi-déflecteur interne,
- ladite paroi-déflecteur interne est située entre l'élément de conduit et la paroi externe du couvercle, et
- ladite chambre de sortie est en communication fluidique avec le volume interne de la cuve et comprend en outre un orifice de sortie de gaz, et une ou plusieurs fenêtres d'entrée de gaz mettant en communication fluidique ladite chambre de sortie de gaz avec le volume interne de la cuve, la ou lesdites fenêtres d'entrée de gaz étant situées entre la paroi-déflecteur et la paroi externe du couvercle.

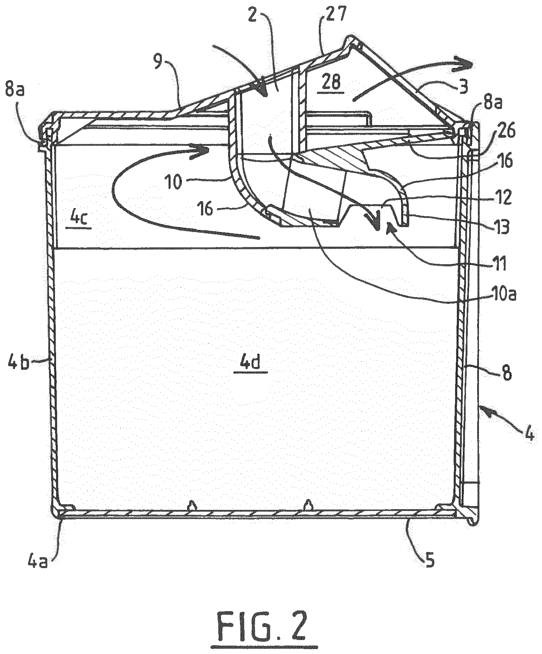

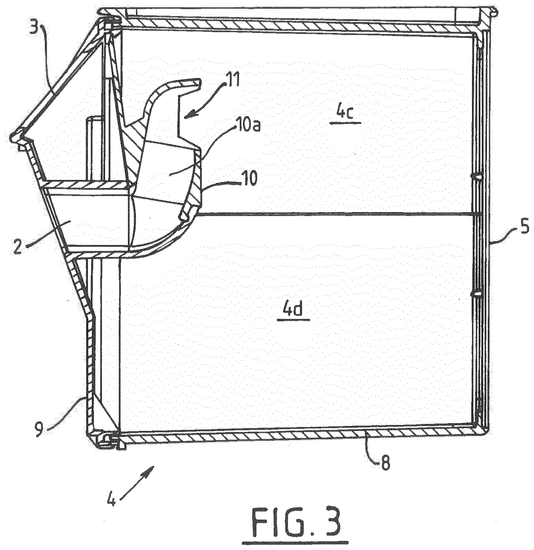

Selon le cas, le réservoir de l'invention peut comprendre l'une ou plusieurs des caractéristiques techniques suivantes :
- l'extrémité libre de l'élément de conduit est munie d'une bouche de sortie dirigée vers le fond de la cuve, c'est-à-dire que l'extrémité libre de l'élément de conduit est recourbée vers le fond de la cuve, donc dirigée vers la surface de l'eau contenue dans la cuve.
- l'extrémité libre est munie d'une bouche de sortie débouchant dans le volume interne de la cuve, en particulier dans le ciel gazeux surmontant l'eau contenue dans la cuve.
- le couvercle est amovible, c'est-à-dire fixé de manière détachable à la paroi périphérique de la cuve à eau.
- le couvercle comprend des moyens ou un système de fixation permettant de le solidariser à la paroi périphérique de la cuve, par exemple un système à emboitement.
- le couvercle et/ou la cuve à eau est en polymère, par exemple en plastique du type polypropylène (PP).
- la cuve à eau est parallélépipédique, c'est-à-dire de section carrée ou rectangulaire, de préférence rectangulaire.
- la cuve à eau est un parallélépipède rectangle.
- l'élément de conduit comprend un ou plusieurs coudes.
- l'élément de conduit comprend deux coudes.
- l'élément de conduit est fixé solidairement au reste du couvercle, par exemple encliqueté ou emboité.
- l'extrémité libre de l'élément de conduit est munie d'une bouche de sortie comprenant des échancrures.
- l'élément de conduit est tubulaire.
- l'élément de conduit comprend un passage interne de gaz s'étendant entre l'orifice d'entrée de gaz et la bouche de sortie.
- l'élément de conduit comprend un premier tronçon linéaire, un premier tronçon coudé, un second tronçon linéaire et un tronçon coudé successifs.
- la chambre de sortie de gaz est délimitée, en haut, par la paroi externe du couvercle et, en bas, une paroi-déflecteur interne.
- la paroi externe du couvercle forme le plafond de la chambre de sortie de gaz, alors que la paroi-déflecteur forme le sol de la chambre de sortie de gaz.
- la paroi-déflecteur est agencée face à la plaque métallique située dans le fond de la cuve, c'est-à-dire qu'elles peuvent être parallèles ou inclinées l'une par rapport à l'autre.
- la chambre de sortie de gaz comprend plusieurs fenêtres d'entrée de gaz, encore appelées « ouies », mettant en communication fluidique la chambre de sortie de gaz avec le volume interne de la cuve, lesdites fenêtres d'entrée de gaz étant situées entre la paroi-déflecteur et la paroi externe du couvercle, c'est-à-dire de préférence agencée(s) latéralement dans la chambre de sortie de gaz.
- la chambre de sortie de gaz comprend deux fenêtres d'entrée de gaz, de préférence les deux fenêtres d'entrée de gaz sont séparées par l'élément de conduit.
- la bouche de sortie de l'extrémité libre de l'élément de conduit débouche toujours dans le ciel gazeux surmontant l'eau contenue dans la cuve que le réservoir soit en position « verticale » (cf. Figure 2) ou en position «renversée» ou « inclinée » (cf. Figure 3).
- la cuve à eau comprend un fond muni d'une plaque métallique en aluminium, alliage d'aluminium ou acier inoxydable.

L'invention concerne aussi un humidificateur de gaz comprenant un réservoir selon l'invention, de préférence un réservoir amovible, ledit humidificateur de gaz servant à humidifier un gaz provenant d'un appareil d'assistance respiratoire médical, typiquement un ventilateur médical.

L'humidificateur de gaz comprend un élément chauffant en contact avec une plaque métallique qui est elle-même en contact avec une autre plaque métallique située dans le fond de la cuve, lorsque la cuve est agencée dans l'humidificateur.

Par ailleurs, l'invention concerne en outre un ensemble humidificateur/ventilateur médical comprenant un ventilateur médical, c'est-à-dire appareil d'assistance respiratoire médical, équipé d'une micro-soufflante électrique et un humidificateur selon l'invention, ledit humidificateur de gaz servant à humidifier le gaz, typiquement de l'air, fourni par la micro-soufflante électrique grâce à de la vapeur d'eau générée dans humidificateur par chauffage au moyen de l'élément chauffant et des deux plaques métalliques.

L'invention va maintenant être mieux comprise grâce à la description détaillée suivante, faite à titre illustratif mais non limitatif, en référence aux figures annexées parmi lesquelles :
- la Figure 1 schématise un ensemble ventilateur/humidificateur de gaz médical,
- la Figure 2 est une vue en coupe longitudinale d'un mode de réalisation d'un réservoir d'eau selon l'invention, représenté en position verticale,
- la Figure 3 montre le réservoir de la Figure 2 rempli d'eau, en position inclinée, et
- la Figure 4 schématise un mode de réalisation du couvercle du réservoir des Figures 2 et 3.

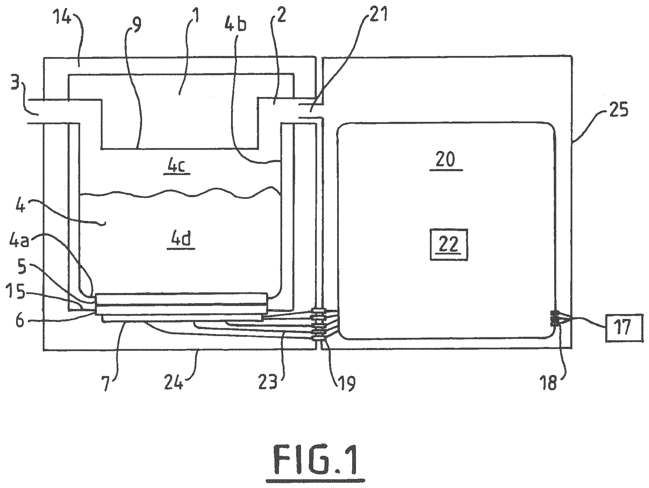

La Figure 1 schématise un mode de réalisation d'un ensemble ventilateur médical/humidificateur de gaz médical comprenant un ventilateur médical 25, c'est-à-dire un appareil de fourniture de gaz d'assistance respiratoire, aussi appelé appareil d'assistance respiratoire, et un humidificateur de gaz 24 en communication fluidique (en 21) et électrique (en 19) l'un avec l'autre.

Plus précisément, le ventilateur médical 25 comprend des moyens de fourniture de gaz, telle une micro-soufflante électrique, encore appelée « turbine » ou « compresseur », permettant de fournir un flux gazeux, par exemple de l'air, à l'humidificateur 24, et des moyens de commande 20, 22, à savoir une carte électronique 20 à (micro)processeur 22. Le flux de gaz délivré par les moyens de fourniture de gaz du ventilateur médical 25 est acheminé, via un circuit de gaz interne du ventilateur comprenant un ou plusieurs conduits de gaz ou analogue, jusqu'à une sortie de gaz 21, tel un connecteur pneumatique, assurant une communication fluidique entre le ventilateur médical 25 et l'humidificateur 24 de gaz.

Par ailleurs, l'humidificateur de gaz 24 comprend un boitier 14 définissant un volume ou logement interne 1 destiné à recevoir un réservoir à eau 4 comprenant un bac ou cuve 8 destiné à contenir de l'eau 4d servant à humidifier le flux de gaz provenant du ventilateur 25. La cuve 8 comprend un couvercle 9, de préférence amovible, fermant le haut de la cuve, comme visible sur la Figure 2.

La cuve 8 du réservoir à eau 4 comprend une entrée 2 de gaz sec par laquelle le gaz à humidifier provenant du ventilateur 25 pénètre dans le réservoir à eau 4 et une sortie 3 de gaz humidifié par laquelle le gaz humidifié au sein de la cuve 8 ressort du réservoir à eau 4 et est ensuite envoyé vers le patient, via une conduite de gaz flexible par exemple reliée à une interface patient de distribution de gaz, tel un masque respiratoire ou analogue.

L'humidificateur de gaz 24 comprend en outre un élément chauffant 7 et une première plaque métallique 6 qui sont agencés dans le fond 15 du boitier de l'humidificateur 24. L'élément chauffant 7 de l'humidificateur de gaz 24 est commandé par les moyens de commande 20, 22 du ventilateur médical 25 et est par ailleurs en contact avec la première plaque métallique 6 de manière à chauffer la première plaque métallique 6 en réponse à un signal de commande de chauffe délivré par les moyens de commande 20, 22 du ventilateur médical 25.

Comme visible sur la Figure 1, le réservoir à eau 4 est agencé dans le boitier 14 de l'humidificateur de gaz 24. La cuve du réservoir 4 comprend une seconde plaque métallique 5 qui est elle-même en contact avec la première plaque métallique 6 du boitier 14 de manière à ce que les calories générées par l'élément chauffant 7 et transmises à la première plaque métallique 6 puissent passer ensuite à la seconde plaque métallique 5, puis à l'eau 4d de la cuve 8 de sorte de chauffer cette eau.

Plus précisément, la seconde plaque métallique 5 est agencée dans le fond 4a de la cuve 8 du réservoir 4, lequel fond 4a définit avec la paroi périphérique 4b de la cuve 8 du réservoir 4, un volume interne 4c contenant l'eau 4d à chauffer qui sert à humidifier le gaz fourni par le ventilateur médical 25. L'eau 4d contenue dans le réservoir 4 est donc vaporisée par chauffage au contact de la seconde plaque métallique 5.

Les plaques métalliques 5, 6 sont par exemple en aluminium, en alliage d'aluminium ou en acier inoxydable.

Le ventilateur médical 25 est alimenté en courant électrique par une alimentation 17 en courant électrique, par exemple le secteur délivrant un courant ayant une tension entre 110 et 230 V. Le courant fourni par la source de courant électrique 17 est acheminé par un câble électrique venant se raccorder électriquement au ventilateur 25 au niveau d'un connecteur électrique amont 18. Le ventilateur médical 25 est quant à lui raccordé électriquement à l'humidificateur de gaz 24 par un connecteur électrique aval 19 ou analogue. Le courant électrique est ensuite acheminé depuis le connecteur électrique aval 19 jusqu'à l'élément chauffant 7 par un ou plusieurs câbles électriques 23.

Selon la présente invention, afin d'éviter ou de limiter les sorties intempestives d'eau du réservoir 4 de l'humidificateur 24, en particulier les retours d'eau par l'entrée de gaz 2 et le conduit 21, en direction de la micro-soufflante électrique du ventilateur médical 25, le couvercle 9 fermant le haut de la cuve 8 a été configuré de façon particulière.

Plus précisément, le couvercle 9 comprend un orifice d'entrée 2 de gaz en communication fluidique avec un élément de conduit creux, c'est-à-dire tubulaire.

L'élément de conduit 10 se projette dans le volume interne 4c de la cuve 8 en direction de la paroi périphérique 4b de la cuve 8 à eau, et comprend par ailleurs une extrémité libre 13 munie d'une bouche de sortie 11, de préférence dirigée vers le fond 4a de la cuve 8.

L'élément de conduit 10 est traversé par un passage interne de gaz 10a s'étendant entre l'orifice d'entrée de gaz 2 et la bouche de sortie 11, comme illustré en Figure 2.

L'élément de conduit 10 peut être formé d'une seule pièce, par exemple par moulage, avec le reste du couvercle 9, notamment en matériau polymère, ou alors il peut être fixé solidairement au reste du couvercle 9 par exemple par emboitement ou encliquetage.

Le couvercle 9 agencé sur la cuve 8 est rendu solidaire de la paroi périphérique 4b de la cuve 8, grâce à un système de fixation adapté, par exemple un système par emboitement ou analogue. Le système de fixation assure aussi une étanchéité fluidique entre le rebord périphérique inférieur 9a du couvercle 9 et le rebord périphérique supérieur 8a de la cuve 8. A cette fin, le rebord périphérique inférieur 9a du couvercle 9 et le rebord périphérique supérieur 8a de la cuve 8 viennent préférentiellement s'emboiter l'un dans l'autre.

Dans le mode de réalisation des Figures 1-4, la cuve 8 à eau est ici un parallélépipède rectangle. Elle comprend donc 4 parois se faisant face 2 à 2, i.e. parallèles 2 à 2 et perpendiculaires 2 à 2, incluant deux petites parois et deux grandes parois, les petites parois ayant une largeur inférieure à celle des grandes parois, la hauteur des petites et des grandes parois étant égale. De préférence, l'élément de conduit 10 se projette dans le volume interne 4c de la cuve 8 en direction de l'une des petites parois de la paroi périphérique 4b de ladite cuve 8 de section rectangulaire.

Comme illustré en Figures 2 à 4, l'élément de conduit 10 est coudé 16, c'est-à-dire que l'élément de conduit 10 comprend un premier tronçon linéaire avec l'orifice d'entrée de gaz 2, un premier tronçon coudé, un second tronçon linéaire et un tronçon coudé situé à l'extrémité libre 13 et portant une bouche de sortie 11, qui sont successifs et traversés par le passage interne 10a (cf. Fig. 2)

L'extrémité libre 13 de l'élément de conduit 10 est ainsi munie d'une bouche de sortie 11, par laquelle arrive le gaz, tel de l'air, provenant de la micro-soufflante, la bouche de sortie 11 débouchant dans le ciel gazeux surmontant l'eau 4d contenue dans le volume interne 4c de la cuve 8, de préférence la bouche de sortie 11 est dirigée vers le fond 4a de la cuve 8.

En effet, une cuve 8 contient un volume maximum d'eau 4d, encore appelé « niveau haut », habituellement figuré par un marquage porté par la cuve 8 permettant à l'utilisateur de ne pas remplir le réservoir 4 avec trop d'eau 4d de sorte de ne pas détériorer les performances de l'humidificateur 24. Les Figures 2 et 3 représentent ainsi la cuve 8 remplie à son volume maximum d'eau 4d.

Comme on le voit, selon l'invention, l'extrémité libre 13 munie de la bouche de sortie 11 débouche toujours dans le ciel gazeux surmontant l'eau 4d contenue dans la cuve 8 que le réservoir 4 soit en position « verticale » de fonctionnement habituel comme représenté en Figure 2, ou en position « renversée » ou « inclinée » comme représenté en Figure 3.

Ainsi, de l'eau ne peut pas sortir intempestivement par l'orifice 2 et aller détériorer la micro-soufflante du ventilateur 25, même lorsque l'humidificateur 25, donc le réservoir 4, est renversé par accident (cf. Figure 3). On comprend immédiatement au vu de la Figure 3, qu'en l'absence de l'élément de conduit 10 de l'invention, de l'eau 4d pourrait sortir du réservoir 4 par l'orifice 2 d'entrée de gaz puisque le niveau de l'eau s'élève au-dessus dudit orifice d'entrée 2.

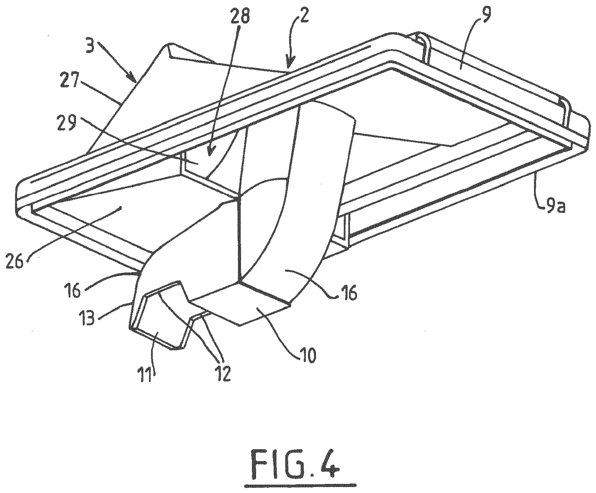

Avantageusement, la bouche de sortie 11 de l'élément de conduit 10 comprend aussi des échancrures 12 et forme dès lors une sorte de « bec », comme illustré en Figure 4 notamment.

La présente invention repose donc sur une intégration, sur le réservoir 4 de l'humidificateur 24, d'un couvercle 9 amélioré qui empêche l'eau de sortir du réservoir 4 par l'orifice d'entrée de gaz 2, ce qui limite ou empêche les remontées d'eau vers le ventilateur 25 et surtout vers la micro-soufflante dudit ventilateur 25 raccordé fluidiquement à l'humidificateur 24 équipé dudit réservoir 4 à couvercle 9 amélioré.

Par ailleurs, le couvercle 9 comprend aussi une chambre de sortie de gaz 28 délimitée, en haut, par la paroi externe 27 du couvercle 9 qui forme alors au moins une partie du plafond de ladite chambre de sortie de gaz 28, et une paroi-déflecteur 26 interne, en bas, qui forme alors au moins une partie du sol de ladite chambre de sortie de gaz 28. La chambre de sortie 28 est en communication fluidique avec le volume interne 4c de la cuve 8 du réservoir 4 et comprend en outre un orifice de sortie de gaz 3 par lequel le gaz humidifié peut être évacué vers le patient.

La paroi-déflecteur 26 interne est située entre l'élément de conduit 10 et la paroi externe 27 du couvercle 9, la paroi-déflecteur 26 étant positionnée face à la plaque métallique 5 située dans le fond 4a de la cuve 9 du réservoir 4.

La chambre de sortie de gaz 28 comprend aussi une ou plusieurs fenêtres d'entrée de gaz 29, appelées « ouïes », mettant en communication fluidique la chambre de sortie de gaz 28 avec le volume interne 4c de la cuve 8, c'est-à-dire son ciel gazeux, même lorsque la cuve 8 est en positionnée « inclinée » ou « renversée » (Fig. 3), la ou lesdites ouïes 29 étant situées entre la paroi-déflecteur 26 et la paroi externe 27 du couvercle 9, comme visible en Fig. 4.

La paroi-déflecteur 26 interne du couvercle 9 constitue donc une sorte de plateforme située sous la sortie de gaz 3, qui permet d'éviter que de l'eau s'échappe directement vers la sortie de gaz 3, notamment en cas de débit d'air important provoquant des vagues à la surface de l'eau 4d.

En outre, les échancrures 12 de la bouche de sortie 11 de l'élément de conduit 10 en forme de bec, en élargissant le cône du flux d'air sortant par ladite bouche de sortie 11 et frappant la surface de l'eau, permettent de limiter l'amplitude des vagues se formant à la surface de l'eau, en particulier lorsque l'eau est au « niveau haut » ou proche de ce niveau, et donc de limiter la quantité d'eau rejetée vers le circuit patient, en fonctionnement normal de l'humidificateur 24.

Un réservoir 4 d'eau amovible selon l'invention est destiné à équiper un humidificateur de gaz 24 d'un ensemble humidificateur 24/ventilateur médical 25 dont le ventilateur 25 est équipé d'une micro-soufflante électrique alimentant l'humidificateur 24 en gaz à humidifier.

## Revendications

1. Réservoir (4) pour humidificateur de gaz (24) chauffant servant à l'humidification de gaz issu d'un appareil d'assistance respiratoire médical comprenant une cuve (8) à eau comprenant une paroi périphérique (4b) et un fond (4a) muni d'une plaque métallique (5), et un couvercle (9) agencé sur la cuve (8) et solidaire de la paroi périphérique (4b) de ladite cuve (8), le couvercle (9) comprenant en outre un orifice d'entrée (2) de gaz en communication fluidique avec un élément de conduit (10), l'élément de conduit (10) se projetant dans le volume interne (4c) de la cuve (8) en direction de la paroi périphérique (4b) de ladite cuve (8), et comprenant une extrémité libre (13) munie d'une bouche de sortie (11), **caractérisé en ce que** :
- le couvercle (9) comprend en outre une chambre de sortie de gaz (28) délimitée par la paroi externe (27) du couvercle (9) et une paroi-déflecteur (26) interne,
- ladite paroi-déflecteur (26) interne est située entre l'élément de conduit (10) et la paroi externe (27) du couvercle (9), et
- ladite chambre de sortie (28) est en communication fluidique avec le volume interne (4c) de la cuve (8) et comprend en outre un orifice de sortie de gaz (3), et une ou plusieurs fenêtres d'entrée de gaz (29) mettant en communication fluidique ladite chambre de sortie de gaz (28) avec le volume interne (4c) de la cuve (8), la ou lesdites fenêtres d'entrée de gaz (29) étant situées entre la paroi-déflecteur (26) et la paroi externe (27) du couvercle (9).

2. Réservoir (4) selon la revendication précédente, **caractérisé en ce que** l'extrémité libre (13) de l'élément de conduit (10) est munie d'une bouche de sortie (11) débouchant dans le ciel gazeux surmontant l'eau (4d) contenue dans le volume interne (4c) de la cuve (8).

3. Réservoir (4) selon l'une des revendications précédentes, **caractérisé en ce que** la bouche de sortie (11) est dirigée vers le fond (4a) de la cuve (8).

4. Réservoir (4) selon l'une des revendications précédentes, **caractérisé en ce que** la cuve (8) à eau est parallélépipédique, de préférence un parallélépipède rectangle.

5. Réservoir (4) selon l'une des revendications précédentes, **caractérisé en ce que** l'élément de conduit (10) comprend un passage interne de gaz (10a) s'étendant entre l'orifice d'entrée de gaz (2) et la bouche de sortie (11).

6. Réservoir (4) selon l'une des revendications précédentes, **caractérisé en ce que** la bouche de sortie (11) comprend des échancrures (12).

7. Réservoir (4) selon l'une des revendications précédentes, **caractérisé en ce que** le couvercle (9) est fixé de manière détachable à la paroi périphérique (4b) de la cuve à eau (8).

8. Réservoir (4) selon la revendication 7, **caractérisé en ce que** la paroi-déflecteur (26) est agencée face à la plaque métallique (5) située dans le fond (4a) de la cuve (8).

9. Réservoir (4) selon la revendication 7, **caractérisé en ce que** la chambre de sortie de gaz (28) comprend plusieurs fenêtres d'entrée de gaz (29).

10. Réservoir (4) selon l'une des revendications précédentes, **caractérisé en ce que** la bouche de sortie (11) de l'extrémité libre (13) de l'élément de conduit (10) débouche toujours dans le ciel gazeux surmontant l'eau (4d) contenue dans la cuve (8) que le réservoir (4) soit en position « verticale » ou en position « renversée » ou « inclinée ».

11. Humidificateur de gaz (24) chauffant servant à l'humidification de gaz issu d'un appareil d'assistance respiratoire médical, comprenant un réservoir (4) selon l'une des revendications précédentes, de préférence un réservoir (4) amovible.

12. Ensemble humidificateur/ventilateur médical (24, 25) comprenant un ventilateur (25) équipé d'une micro-soufflante électrique et un humidificateur (24) selon la revendication 11.

## Patentansprüche

1. Behälter (4) für einen beheizten Gasbefeuchter (24), der zur Befeuchtung eines Gases dient, das aus einer medizinischen Beatmungseinrichtung stammt, die einen Wassertank (8) umfasst, das eine umlaufende Wand (4b) und einen Boden (4a) umfasst, der mit einer Metallplatte (5) versehen ist, und einen Deckel (9), der auf dem Tank (8) angeordnet ist und mit der umlaufenden Wand (4b) des Tanks (8) fest verbunden ist, wobei der Deckel (9) weiter eine Gaseingangsöffnung (2) in Fluidkommunikation mit einem Kanalelement (10) umfasst, wobei das Kanalelement (10) in das interne Volumen (4c) des Tanks (8) in Richtung der umlaufenden Wand (4b) des Tanks (8) vorspringt, und ein freies Ende (13) umfassend, das mit einem Ausgangsdurchlass (11) versehen ist, **dadurch gekennzeichnet, dass**:
- der Deckel (9) weiter eine Gasausgangskammer (28) umfasst, die durch die externe Wand (27) des Deckels (9) und einen internen Wand-Abweiser (26) eingegrenzt wird,
- wobei sich der interne Wand-Abweiser (26) zwischen dem Kanalelement (10) und der externen Wand (27) des Deckels (9) befindet, und
- die Ausgangskammer (28) in Fluidkommunikation mit dem internen Volumen (4c) des Tanks (8) ist und weiter eine Gasausgangsöffnung (3) und ein oder mehrere Gaseingangsfenster (29) umfasst, die die Gasausgangskammer (28) mit dem internen Volumen (4c) des Tanks (8) in Fluidkommunikation bringt, wobei sich das oder die Gaseingangsfenster (29) zwischen dem Wand-Abweiser (26) und der externen Wand (27) des Deckels (9) befinden.

2. Behälter (4) nach dem vorstehenden Anspruch, **dadurch gekennzeichnet, dass** das freie Ende (13) des Kanalelements (10) mit einem Ausgangsdurchlass (11) versehen ist, der in die gasförmige Glocke mündet, die das Wasser (4d) überragt, das in dem internen Volumen (4c) des Tanks (8) enthalten ist.

3. Behälter (4) nach einem der vorstehenden Ansprüche, **dadurch gekennzeichnet, dass** der Ausgangsdurchlass (11) zum Boden (4a) des Tanks (8) gerichtet ist.

4. Behälter (4) nach einem der vorstehenden Ansprüche, **dadurch gekennzeichnet, dass** der Wassertank (8) quaderförmig, vorzugsweise ein quaderförmiges Rechteck ist.

5. Behälter (4) nach einem der vorstehenden Ansprüche, **dadurch gekennzeichnet, dass** das Kanalelement (10) einen internen Gasdurchgang (10a) umfasst, der sich zwischen der Gaseingangsöffnung (2) und dem Ausgangsdurchlass (11) erstreckt.

6. Behälter (4) nach einem der vorstehenden Ansprüche, **dadurch gekennzeichnet, dass** der Ausgangsdurchlass (11) Ausschnitte (12) umfasst.

7. Behälter (4) nach einem der vorstehenden Ansprüche, **dadurch gekennzeichnet, dass** der Deckel (9) in abnehmbarer Form an der umlaufenden Wand (4b) des Wassertanks (8) fixiert ist.

8. Behälter (4) nach Anspruch 7, **dadurch gekennzeichnet, dass** der Wand-Abweiser (26) gegenüber der Metallplatte (5) angeordnet ist, die sich am Boden (4a) des Tanks (8) befindet.

9. Behälter (4) nach Anspruch 7, **dadurch gekennzeichnet, dass** die Gasausgangskammer (28) mehrere Gaseingangsfenster (29) umfasst.

10. Behälter (4) nach einem der vorstehenden Ansprüche, **dadurch gekennzeichnet, dass** der Ausgangsdurchlass (11) des freien Endes (13) des Kanalelements (10) immer in die gasförmige Glocke mündet, die das Wasser (4d) überragt, das in dem Tank (8) enthalten ist, unabhängig davon, ob der Behälter (4) in "vertikaler" Position oder in "gestürzter" oder "geneigter" Position ist.

11. Beheizter Gasbefeuchter (24), der zur Befeuchtung eines Gases dient, das aus einer medizinischen Beatmungseinrichtung stammt, einen Behälter (4) nach einem der vorstehenden Ansprüche, vorzugsweise einen abnehmbaren Behälter (4), umfassend.

12. Medizinische Befeuchter-/Lüfterbaugruppe (24, 25), umfassend einen Lüfter (25), der mit einem elektrischen Mikrogebläse und einem Befeuchter (24) nach Anspruch 11 ausgerüstet ist.

## Claims

1. Tank (4) for a heated gas humidifier (24) used to humidify gas coming from a medical breathing assistance apparatus comprising a water tank (8) comprising a peripheral wall (4b) and a bottom (4a) provided with a metal plate (5), and a cover (9) arranged on the tank (8) and integral with the peripheral wall (4b) of said tank (8), with the cover (9) further comprising a gas inlet orifice (2) in fluid communication with a conduit element (10), with the conduit element (10) projecting into the inner volume (4c) of the tank (8) in the direction of the peripheral wall (4b) of said tank (8), and comprising a free end (13) provided with an outlet mouth (11), **characterised in that**:
- the cover (9) further comprises a gas outlet chamber (28) delimited by the outer wall (27) of the cover (9) and an inner deflector-wall (26),
- said inner deflector-wall (26) is located between the conduit element (10) and the outer wall (27) of the cover (9), and
- said outlet chamber (28) is in fluid communication with the inner volume (4c) of the tank (8) and further comprises a gas outlet orifice (3), and one or more gas inlet windows (29) that put into fluid communication said gas outlet chamber (28) with the inner volume (4c) of the tank (8), said gas inlet window(s) (29) being located between the deflector-wall (26) and the outer wall (27) of the cover (9).

2. Tank (4) according to the preceding claim, **characterised in that** the free end (13) of the conduit element (10) is provided with an outlet mouth (11) that opens into the expansion space surmounting the water (4d) contained in the inner volume (4c) of the tank (8).

3. Tank (4) according to one of the preceding claims, **characterised in that** the outlet mouth (11) is directed towards the bottom (4a) of the tank (8).

4. Tank (4) according to one of the preceding claims, **characterised in that** the water tank (8) is parallelepiped, preferable a rectangular parallelepiped.

5. Tank (4) according to one of the preceding claims, **characterised in that** the conduit element (10) comprises an internal gas passage (10a) that extends between the gas inlet orifice (2) and the outlet mouth (11).

6. Tank (4) according to one of the preceding claims, **characterised in that** the outlet mouth (11) comprises notches (12).

7. Tank (4) according to one of the preceding claims, **characterised in that** the cover (9) is removably fastened to the peripheral wall (4b) of the water tank (8).

8. Tank (4) according to claim 7, **characterised in that** the deflector-wall (26) is arranged facing the metal plate (5) located in the bottom (4a) of the tank (8).

9. Tank (4) according to claim 7, **characterised in that** the gas outlet chamber (28) comprises several gas inlet windows (29).

10. Tank (4) according to one of the preceding claims, **characterised in that** the outlet mouth (11) of the free end (13) of the conduit element (10) always opens into the expansion space surmounting the water (4d) contained in the tank (8) whether the tank (4) is in the "vertical" position or in the "inverted" or "inclined" position.

11. Heated gas humidifier (24) used for the humidification of gas coming from a medical breathing assistance apparatus, comprising a tank (4) according to one of the preceding claims, preferably a tank (4) that can be removed.

12. Medical humidifier/ventilator unit (24, 25) comprising a ventilator (25) provided with an electric micro-fan and a humidifier (24) according to claim 11.
